# EUROPEAN PATENT SPECIFICATION

(11) **EP 2 916 896 B1**
(45) Date of publication and mention of the grant of the patent: **19.04.2017**
(21) Application number: 13788968.9
(22) Date of filing: 06.11.2013
(51) Int. Cl.: A61M 15/00

(54) **ASSEMBLY FOR AN INHALATION DEVICE AND USE OF A SEALING MEMBER**
ANORDNUNG FÜR EINE INHALATIONSVORRICHTUNG UND VERWENDUNG EINES DICHTELEMENTS
ENSEMBLE POUR DISPOSITIF D'INHALATION ET UTILISATION D'UN ÉLÉMENT D'ÉTANCHÉITÉ

(30) Priority: 12.11.2012 EP 12192242
(43) Date of publication of application: 16.09.2015
(73) Proprietor: Sanofi SA, 1214 Vernier (CH)
(72) Inventor: MAYER, Stefan, 79098 Freiburg Im Breisgau (DE); DOEDENS, Lucas, 80337 München (DE); PENGILLEY, Roy, 81373 München (DE)
(74) Representative: Zwicker, Jörk
(86) International application number: PCT/EP2013/073186
(87) International publication number: WO 2014/072352

(56) References cited:
- US-A1- 2003 136 405
- US-A1- 2008 185 000
- US-A1- 2009 056 710
- US-A1- 2009 260 626

## Description

The disclosure relates to an assembly for an inhalation device. Furthermore, the disclosure relates to the use of the assembly in an inhalation device.

An inhalation device is described in document WO 2009/065707 A1. A seal is disclosed in US 2009/260626 A1. US 2003/136405 A1 discloses a pharmaceutical powder partridge.

It is an object of the present disclosure to provide an assembly for an inhalation device comprising improved properties. Furthermore, it is an object of the present disclosure to provide a use of a sealing member for mechanical cooperation with a metering element of an inhalation device.

This object may be achieved by the subject matter of the independent claims. Advantageous embodiments and refinements are the subject matter of the dependent claims.

According to a first aspect of the invention, an assembly for an inhalation device is provided. The assembly comprises a storage chamber configured to hold a quantity of a substance, a metering element configured to be moved in a moving direction with respect to the storage chamber, and a sealing member mechanically cooperating with the metering element, wherein the sealing member comprises an opening which receives the metering element, and wherein the sealing member comprises at least one sealing lip which is deflectable in a direction corresponding to the moving direction of the metering element.

The inhalation device may be used by a user for inhaling a powdery substance. The substance may contain a medicament, for example a medicament for treating asthma.

The metering element may comprise a longitudinal axis. The moving direction of the metering element may be a direction along the longitudinal axis of the metering element. The metering element may be configured to be rotated and axially moved with respect to the storage chamber along the longitudinal axis of the metering element. In case that the metering element performs a combined axial and rotational movement, the direction of the axial movement may be regarded as the moving direction. Alternatively, the direction of the rotational movement or the direction of the combined rotational and axial movement may be regarded as the moving direction. The metering element may be configured as a rod. The metering element may have a rectangular cross-section. Alternatively, the metering element may have a circular or elliptical cross-section.

The metering element may comprise a metering chamber. The metering chamber may be a cavity in the metering element. The cavity may be conical. The metering chamber may be configured to hold a sub-quantity of substance. The sub-quantity of substance may be retrieved from the storage chamber by means of the metering chamber when the metering element is moved in a dosing direction with respect to the storage chamber. The dosing direction may be a direction towards a dispensing end of the device. In particular, for delivering a dose of substance, the metering element may be moved along the longitudinal axis towards a dispensing end of the device. After use, the metering element may be moved away from the dispensing end of the device.

The sealing member may comprise a main part, wherein the at least one sealing lip is connected to the main part. In particular, the at least one sealing lip and the main part may be formed unitarily. For example, the sealing member may be an injection moulded part.

Due to the cooperation of the sealing member and the metering element, the sealing member may seal the storage chamber. Preferably, the sealing lip directly contacts the metering element. In particular, the at least one sealing lip may exert a force on the metering element. In particular, the sealing lip may be manufactured with an oversize, such that the sealing lip may be compressed by the presence of the metering element. Thereby, it may be ensured that the sealing lip tightly abuts the metering element. Thereby, the sealing lip may seal the storage chamber such that no moisture or dirt may intrude into the storage chamber. Furthermore, manufacturing tolerances of the metering element may be compensated by the sealing lip. In particular, the sealing lip may be configured to mechanically cooperate with different metering elements having different dimensions due to manufacturing tolerances. In particular, the size of the sealing lip may be such that it may reliably seal the storage chamber. Thereby, a reliable inhalation device may be provided.

According to a further embodiment, the at least one sealing lip may be adapted and arranged for removing excess powdery substance from the metering element when the metering element is moved in a dosing direction with respect to the storage chamber, in particular in a direction towards the distal end of the device. Thereby, a high dosing accuracy may be achieved. In particular, dispense of an overdose to a user may be inhibited.

The at least one sealing lip may adhere to the metering element due to a friction force. When the metering element is moved, the sealing lip may be deflected in a direction corresponding to the moving direction of the metering element. Thereby, an abrasion of the sealing lip by the movement of the metering element may be diminished.

Furthermore, when the metering element is moved in the dosing direction, the metering chamber may pass the sealing lip on its way from the storage chamber towards the dispensing end of the device. The sealing lip may inhibit powdery substance falling out of the metering chamber before it reaches the powder channel. Thereby, dispense of a too little amount of substance to a user may be inhibited.

According to a preferred embodiment, the transverse section of the at least one sealing lip comprises the shape of a trapezoid. The narrower end of the trapezoid may be directed towards the metering element.

The at least one sealing lip may be prevented from protruding into the metering chamber when the metering chamber passes the sealing lip. Thereby, removal of substance from the metering chamber upon mechanical cooperation with the sealing lip may be prevented. In particular, the dimensions of the sealing lip may be adapted to the dimensions of the metering chamber, such that the sealing lip may be prevented from protruding into the metering chamber. Also a trapezoidal shape of the sealing lip may help to prevent the sealing lip from protruding into the metering chamber. Thereby, a high dosing accuracy may be achieved.

Preferably, the at least one sealing lip comprises a top surface and a bottom surface. The top surface may decline in a direction towards a proximal end of the device in a viewing direction towards the metering element. Alternatively, the top surface may run perpendicular with respect to the longitudinal axis of the metering element. The bottom surface may rise in a direction towards a proximal end of the device in a viewing direction towards the metering element. Alternatively, the bottom surface may run perpendicular with respect to the longitudinal axis of the metering element.

In one embodiment, the opening of the sealing member may have an elongated form in plan view onto the opening. In particular, the opening may have a rectangular form. Alternatively, the opening may have a circular or elliptical form. The metering element may protrude through the opening of the sealing member. In particular, the metering element may be guided through the opening of the sealing member when the metering element is moved with respect to the storage chamber.

The sealing member may comprise an elastic material. For example, the sealing member may comprise a thermoplastic or rubber material. Due to the elasticity of the material, the sealing lip may be able to deflect in a direction corresponding to the moving direction of the metering element.

According to a preferred embodiment, the at least one sealing lip is disposed circumferentially around the metering element. In particular, the metering element may be completely enclosed by the sealing lip. In particular, no gap may occur between the sealing member, respectively the sealing lip, and the metering element.

The at least one sealing lip may be adapted to provide a constant force on the metering element at any position of the metering element with respect to the storage chamber when the sealing member and the metering element mechanically cooperate with one another. This may be achieved due to the sealing lip being deflectable in a direction corresponding to the moving direction of the metering element.

According to one embodiment, the sealing member comprises a plurality of sealing lips. For example, the sealing member may comprise two sealing lips. Alternatively, the sealing member may comprise more than two sealing lips, for example three or four sealing lips. Thereby, the sealing function of the sealing member may be improved, compared to a sealing member with only one sealing lip. Preferably, each sealing lip is configured as described above.

The sealing lips may be arranged one above another in a direction along the longitudinal axis of the metering element.

The sealing member may comprise at least one cavity which may be arranged between two adjacent sealing lips. When the metering element is moved towards a dispensing end of the device, excess powdery substance may be gathered in the cavity.

According to a further aspect of the invention, the use of a sealing member for mechanical cooperation with a metering element of an inhalation device is provided, wherein the sealing member comprises an opening which receives the metering element, and wherein the sealing member comprises at least one sealing lip, and wherein the sealing lip is configured to be deflected in a direction corresponding to the moving direction of the metering element. In particular, the sealing member may be configured as described above.

The term "substance" or "medicament", as used herein may mean a pharmaceutical formulation containing at least one pharmaceutically active compound, for example for the treatment of obstructive airway or lung diseases such as asthma or chronic obstructive pulmonary disease (COPD), local respiratory tract oedema, inflammation, viral, bacterial, mycotic or other infection, allergies, diabetes mellitus.

The active pharmaceutical compound is preferably selected from the group consisting of active pharmaceutical compounds suitable for inhalation, preferably antiallergenic, antihistamine, anti-inflammatory, antitussive agents, bronchodilators, anticholinergic drugs, and combinations thereof.

The active pharmaceutical compound may for example be chosen from:
an insulin such as human insulin, e.g. a recombinant human insulin, or a human insulin analogue or derivative, a glucagon-like peptide (GLP-1) or an analogue or derivative thereof, or exendin-3 or exendin-4 or an analogue or derivative of exendin-3 or exendin-4;
an adrenergic agent such as a short acting β2-agonists (e.g. Salbutamol, Albuterol, Levosalbutamol, Fenoterol, Terbutaline, Pirbuterol, Procaterol, Bitolterol, Rimiterol, Carbuterol, Tulobuterol, Reproterol), a long acting β2-agonist (LABA, e.g. Arformoterol, Bambuterol, Clenbuterol, Formoterol, Salmeterol), an ultra LABA (e.g. Indacaterol) or another adrenergic agent (e.g. Epinephrine, Hexoprenaline, Isoprenaline (Isoproterenol), Orciprenaline (Metaproterenol));
a glucocorticoid (e.g. Beclometasone, Budesonide, Ciclesonide, Fluticasone, Mometasone, Flunisolide, Betamethasone, Triamcinolone);
an anticholinergic agent or muscarinic antagonist (e.g. Ipratropium bromide, Oxitropium bromide, Tiotropium bromide);
a mast cell stabilizer (e.g. Cromoglicate, Nedocromil);
a xanthine derivative (e.g. Doxofylline, Enprofylline, Theobromine, Theophylline, Aminophylline, Choline theophyllinate);
an eicosanoid inhibitor, such as a leukotriene antagonist (e.g. Montelukast, Pranlukast, Zafirlukast), a lipoxygenase inhibitor (e.g. Zileuton) or a thromboxane receptor antagonist (e.g. Ramatroban, Seratrodast);
a phosphodiesterase type-4 inhibitor (e.g. Roflumilast);
an antihistamine (e.g. Loratadine, Desloratadine, Cetirizen, Levocetirizine, Fexofenadine);
an allergen immunotherapy (e.g. Omalizumab);
a mucolytic (e.g. Carbocisteine, Erdosteine, Mecysteine);
an antibiotic or antimycotic;
or a combination of any two, three or more of the above-mentioned compound classes or compounds (e.g. Budesonide/Formoterol, Fluticasone/Salmeterol, Ipratropium bromide/Salbutamol, Mometasone/Formoterol);
or a pharmaceutically acceptable salt or solvate or esters of any of the above named compounds.

Pharmaceutically acceptable salts are for example acid addition salts and basic salts. Acid addition salts are e.g. a chloride, bromide, iodide, nitrate, carbonate, sulfate, methylsulfate, phosphate, acetate, benzoate, benzenesulfonate, fumarate, malonate, tartrate, succinate, citrate, lactate, gluconate, glutamate, edetate, mesylate, pamoate, pantothenate or a hydroxy-naphthoate salt. Basic salts are for example salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology. Pharmaceutically acceptable ester may for example be acetates, propionates, phosphates, succinates or etabonates.

Pharmaceutically acceptable solvates are for example hydrates.

Further features and refinements become apparent from the following description of the exemplary embodiments in connection with the figures.
Figure 1 schematically shows a sectional view of an inhalation device.
Figure 2 schematically shows a cross-sectional view of a metering element.
Figure 3 schematically shows a sealing member cooperating with a metering element of Figure 1 in more detail.
Figure 4 schematically shows an alternative embodiment of a sealing member cooperating with a metering element.

Like elements, elements of the same kind and identically acting elements may be provided with the same reference numerals in the figures.

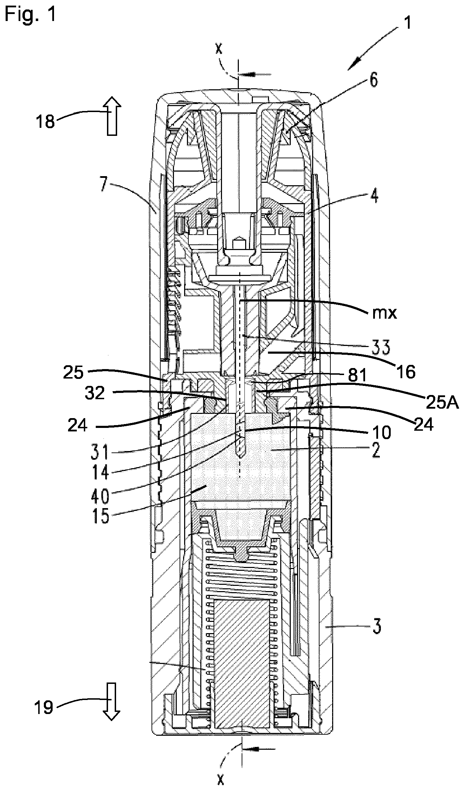

Figure 1 shows a sectional view of an inhalation device 1. The inhalation device 1 is configured to be activated by a suction airflow generated by a user. The inhalation device 1 comprises a housing 3. Furthermore, the device 1 comprises an outer cylinder 4. The outer cylinder 4 is secured against axial movement with respect to the housing 3. The outer cylinder 4 is rotatable with respect to the housing 3.

Furthermore, the inhalation device 1 comprises a mouthpiece 6. Via the mouthpiece 6, air is sucked into the inhalation device 1. The inhalation device 1 further comprises a cap 7. The cap 7 may be configured as a screw cap. The cap 7 is used for covering the mouthpiece 6. The cap 7 may be rotatable about a main longitudinal axis x of the inhalation device 1 in a first direction with respect to the housing for screwing the cap 7 onto the device 1 and in a second direction with respect to the housing 3 for unscrewing the cap 7 from the device 1. The outer cylinder 4 is rotationally connected to the cap 7. In particular, the outer cylinder 4 follows a rotation of the cap 7 with respect to the housing 3. For a detailed description of the components of the inhalation device 1 and their mechanical cooperation it is referred to document WO 2009/065707 A1, in particular as far as the operation of the device 1 is concerned.

The device 1 further comprises a storage chamber 15. The storage chamber 15 holds at least one dose of a substance 2. In particular, the storage chamber 15 may hold a plurality of doses of a substance 2. The substance 2 may comprise a drug. The substance 2 may comprise a powder.

The storage chamber 15 is terminated by a chamber sealing 24. In particular, the side of the storage chamber 15 which is faced towards the mouthpiece is terminated by the chamber sealing 24. The device 1 further comprises a rotary part 25. The rotary part 25 is connected in a rotationally fixed manner to the outer cylinder 4. Accordingly, the rotary part 25 follows a rotation of the outer cylinder and, hence, of the cap 7 about the main longitudinal axis x with respect to the storage chamber 15.

The chamber ceiling 24 comprises a central through opening. A cylindrical portion 25A of the rotary part 25 passes through the central through opening of the chamber ceiling 24.

The inhalation device 1 further comprises a metering element 33. The metering element 33 may comprise a metering rod. The metering element 33 may have a circular or a non-circular cross-section. For example, the metering element 33 may have a rectangular cross-section.

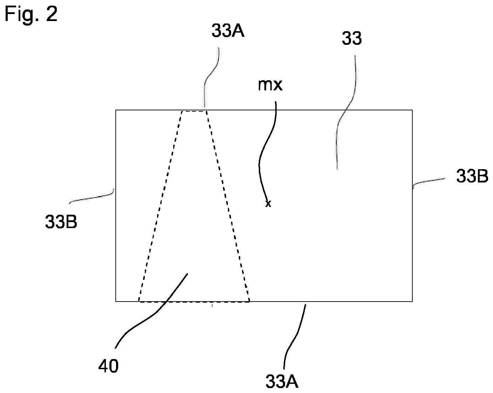

Figure 2 shows a cross-sectional view of the metering element 33 in a plane perpendicular to the longitudinal axis x. As can be seen in Figure 2, the metering element 33 may comprise a first side 33A and a second side 33B. The first side 33A may be broader than the second side 33B. The dimension of the first side 33A may comprise a length from 3 mm to 10 mm. For example, the length of the first side 33A may be 6 mm. The dimension of the second side 33B may comprise a length from 0.5 mm to 3 mm. For example, the dimension of the second side 33B may comprise a length of 1.5 mm.

The metering element 33 comprises a longitudinal axis mx. The longitudinal axis mx of the metering element 33 is parallel to the main longitudinal axis x of the device 1. In particular, the longitudinal axis mx coincides with the main longitudinal axis x of the device 1. The metering element 33 is axially and rotationally movable with respect to the storage chamber 15. When the cap 7 is demounted from the device 1, i.e. during an operation of the device 1, the metering element 33 is moved in a distal direction 18. The distal direction 18 is a direction towards a dispensing end of the device. When the cap 7 is remounted onto the device 1, i.e. after an operation was completed, the metering element 33 is moved in a proximal direction 19. The proximal direction 19 is a direction away from the dispensing end of the device. The metering element 33 is rotationally connected to the rotary part 25 by mechanical cooperation with the rotary part 25. Accordingly, the metering element 33 follows rotational movement of the cap 7 and, hence, of the rotary part 25 about the main longitudinal axis x when the cap 7 is mounted onto the device 1 or demounted from the device 1.

The metering element 33 comprises a metering chamber 40. The metering chamber 40 is located near a proximal end of the metering element 33. The proximal end of the metering element 33 is the end, which his located in the storage chamber 15 when the cap 7 is mounted on the device. The metering element 33 is configured for moving the metering chamber 40 from a first position, wherein the metering chamber 40 is located inside the storage chamber 15, to a second position, wherein the metering 40 is located outside of the storage chamber 15. The metering chamber 40 is configured for measuring and accommodating a sub-quantity 14 of the substance 2 which is to be dispensed during an inhalation action performed by a user. In particular, a sub-quantity 14 of the substance 2 may be transported from the storage chamber 15 to a powder channel 16 via the metering chamber 40. In order to collect a sub-quantity 14 of the substance 2, the metering chamber 40 comprises an opening 10. As can be seen in Figure 2, the opening 10 is arranged eccentric with respect to the axis mx on the metering element 33, in order to achieve an adequate filling of the metering chamber 40 with substance 2. In particular, the metering chamber 40 helicoidally moves through the storage chamber 15, thereby gathering a sub-quantity 14 of substance 2.

For a detailed description of the operation of the metering element 33, it is referred to document WO 2009/065707 A1.

The device 1 shown in Figure 1 further comprises a sealing member 31. The sealing member 31 comprises a main body 32 and a sealing lip 81. The sealing member 31 is arranged in the centre of the cylindrical portion 25A. In particular, the sealing member 31 is arranged adjacent to the cylindrical portion 25 in a radial inward direction. The sealing member 31 is arranged between the cylindrical portion 25A and the metering element 33. The sealing member 31 is arranged circumferentially around the metering element 33. In particular, the sealing lip 81 encloses the metering element 33 completely. The sealing member 31 is arranged at that end of the storage chamber 15 which is faced towards the mouthpiece 6. The sealing member is arranged outside of the storage chamber 15.

The sealing member 31 comprises or consists of a rubber material or a similar elastic material. For example, the sealing member 31 comprises a thermoplastic elastomer. The sealing member 31 may be produced by a two-component injection molding process together with the rotary part 25. Alternatively, the sealing member may be produced in a separate manufacturing step. In the embodiment shown in Figure 1, the sealing member 31 and the rotary part 25 are connected to one another such that the sealing member 31 is secured against axial and rotational movement with respect to the rotary part 25. The sealing member may, for example, be clipped into the rotary part 25.

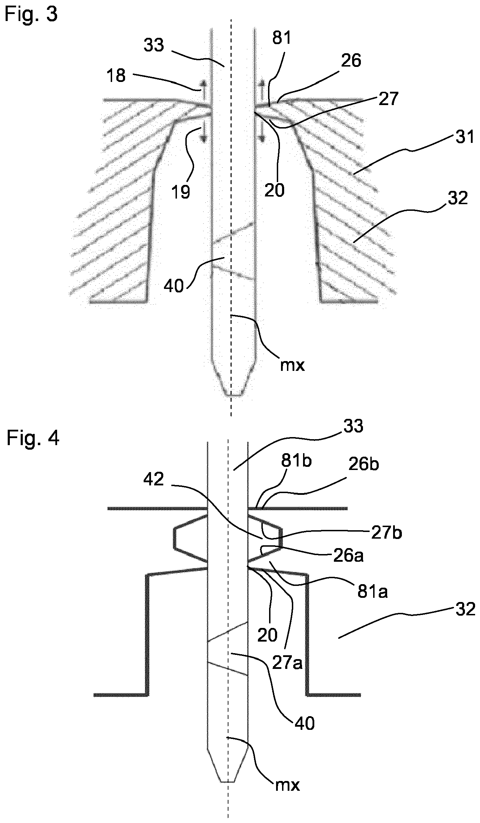

Figure 3 shows the sealing member of Figure 1 in more detail.

The sealing lip 81 may protrude from the main body 32 in a direction which is essentially perpendicular to the longitudinal axis x of the device. In a sectional view, as shown in Figures 1, 3 and 4, the main body 32 extends substantially perpendicular to the extending direction of the sealing lip 81. The main body 32 and the sealing lip 81 are connected to each other, in particular formed unitarily. For example, the sealing member 31 is an injection moulded part. The sealing lip 81 comprises a top surface 26 and a bottom surface 27. The top surface 26 of the sealing lip 81 is the surface which faces towards the mouthpiece 6 of the device. The bottom surface 27 of the sealing lip 81 faces towards the storage chamber 15. The top surface 26 of the sealing lip 81 coincides with a top surface of the main body 32. The top surface 26 declines in a direction towards a proximal end of the device in a viewing direction towards the metering element 33. The bottom surface 27 rises in a direction towards a distal end of the device in a viewing direction towards the metering element 33. The distal end is the dispensing end of the device and the proximal end is the end opposite to the distal end. In particular, the top surface 26 and the lower surface 27 taper in the direction towards the longitudinal axis mx. In particular, the profile of the sealing lip 81 comprises the shape of a truncated cone, wherein the more slender end of the cone is faced towards the metering element 33. In an alternative embodiment, only one of the top surface 26 and the bottom surface 27 may decline, respectively rise, with respect to the metering element 33, while the other one of the top surface 26 and the bottom surface 27 may run perpendicular with respect to the longitudinal axis mx. Thereby, the thickness of the sealing lip 81 may diminish in the direction towards the longitudinal axis mx.

The sealing member 31 is configured for sealing that end of the storage chamber 15 which is closest to the mouthpiece. In particular, the sealing lip 81 is configured to inhibit an intrusion of moisture or dirt into the storage chamber 15 through an opening 20 of the sealing member 31 via which the metering element 33 passes through the sealing member 31 into the storage chamber 15.

The sealing member 31, in particular the sealing lip 81, is in contact with the metering element 33. In particular, it exerts a pressure on the metering element 33 due to an oversize of the sealing member 31. When the metering element 33 starts to move in the distal direction 18 during an operation of the device, the sealing lip 81 may, due to its elasticity and due to adhesion forces which act between the sealing lip 81 and the metering element 33, be deflected in the distal direction 18. The elasticity of the sealing lip 81 and the adhesion forces may cause a reliable sealing of the storage chamber 15. Due to the deflection of the sealing lip 81, an abrasion of the sealing lip 81 may be reduced. When the metering element 33 is further moved in the distal direction 18, the sealing lip 81 may run along the metering element 33. Thereby, the sealing lip 81 may remove excess powdery substance 2 from the metering element 33, which may stick to an outer surface of the metering element 33. Thereby, the dosing accuracy of the inhalation device 1 may be increased. In particular, it may be inhibited that excess powdery substance may be inhaled by a user.

When the metering element 33 is moved in the proximal direction, i. e. after an operation was completed, the sealing lip 81 may be analogously deflected in the proximal direction.

In particular, the sealing lip 81 may be deflected such that the contact force between the sealing lip 81 and the metering element 33 in a radial direction is substantially constant. In particular, the contact force remains substantially constant for different moving directions of the metering element 33.

Due to manufacturing tolerances, different metering elements 33 may comprise different outer dimensions, in particular different dimensions of the first side 33A and the second side 33B. For example, the outer dimensions of different metering elements 33 may differ from one another by up to 1 mm. The sealing member 31, in particular the sealing lip 81, is configured to compensate said manufacturing tolerances. Therefore, the sealing member 31 may be produced with an oversize. In particular, the sealing member 31 is configured to receive and to seal metering elements 33 with dimensions which differ from one another due to manufacturing tolerances. In particular, due to its elasticity, the sealing lip 81 may be compressed in a radial direction by the presence of the metering element such that it may adapt to the outer dimensions of the metering element. For different metering elements 33, in particular for metering elements with different dimensions, the sealing lip 81 exerts a force onto the metering element 33 which is sufficient to seal the storage chamber 15 and to remove excess powdery substance from the metering element 33.

Furthermore, the sealing member 31, in particular the sealing lip 81 may be produced such that manufacturing tolerances of the sealing lip 81 due to shrinking effects which may occur during the manufacturing of the sealing member 31 may be compensated.

Figure 4 shows an alternative embodiment of a sealing member 31. This sealing member comprises a first and a second sealing lip 81 a, 81 b. The sealing lips 81 a, 81 b are arranged one above another in a direction along the longitudinal axis x of the device. A first sealing lip 81 a faces towards the proximal end of the device, while a second sealing lip 81 b faces towards the distal end of the device. Each of the sealing lips 81 a, 81 b may be configured as the sealing lip described in Figure 3. In the embodiment shown in Figure 4, the first sealing lip 81 a comprises a top surface 26a and a bottom surface 27a which are inclined with respect to the metering element 33, respectively the longitudinal axis x of the device. In an alternative embodiment, the top surface 26a and/or the bottom surface 27a of the first sealing lip 81 a may run perpendicular with respect to the metering element 33.The second sealing lip 81 b comprises a top surface 26b which runs perpendicular with respect to the metering element 33. A bottom surface 27b of the second sealing lip 81 b is slightly inclined with respect to the metering element 33. Yet, in an alternative embodiment, the bottom surface 27b of the second sealing lip 81 b may run perpendicular with respect to the metering element 33. In a further alternative embodiment, the top surface 26b of the second sealing lip 81 b may be inclined with respect to the metering element 33. By using two sealing lips 81 a, 81 b instead of only one sealing lip, the sealing function of the sealing member 31 may be further improved. Furthermore, the removal of excess powdery substance from the metering element may be even more reliable.

The sealing member 31 may comprise a cavity 42 arranged between the sealing lips 81. The cavity 42 may receive excess powdery substance which is removed from the metering element 33 by the second sealing lip 81 b.

In an alternative embodiment which is not depicted, the sealing member may comprise more than two sealing lips, for example three or four sealing lips. The sealing lips may be arranged one above another in a direction along the longitudinal axis mx.

### Reference numerals

- 1: inhalation device
- 2: substance
- 3: housing
- 4: outer cylinder
- 6: mouthpiece
- 7: cap
- 14: sub-quantity
- 15: storage chamber
- 16: powder channel
- 18: distal direction
- 19: proximal direction
- 20: opening
- 24: chamber ceiling
- 25: rotary part
- 25A: cylindrical portion
- 26: top surface
- 26a: top surface of first sealing lip
- 26b: top surface of second sealing lip
- 27: bottom surface
- 27a: bottom surface of first sealing lip
- 27b: bottom surface of second sealing lip
- 31: sealing member
- 32: main body
- 33: metering element
- 33A: first side
- 33B: second side
- 40: metering chamber
- 42: cavity
- 81: sealing lip
- 81a: first sealing lip
- 81b: second sealing lip
- x: device axis
- mx: longitudinal axis of metering element
- sx: longitudinal axis of sealing lip

## Claims

1. An assembly for an inhalation device (1), wherein the assembly comprises:
- a storage chamber (15) configured to hold a quantity of a substance (2),
- a metering element (33) configured to be moved in a moving direction with respect to the storage chamber (15), and
- a sealing member (31) mechanically cooperating with the metering element (33), wherein the sealing member (31) comprises an opening (20) which receives the metering element (33), and wherein the sealing member (31) comprises a sealing lip (81) **characterised in that** the sealing lip is deflectable in a direction corresponding to the moving direction of the metering element (33).

2. The assembly according to claim 1,
wherein a transverse section of the sealing lip (81) comprises the shape of a trapezoid.

3. The assembly according to claim 2,
wherein the narrower end of the trapezoid is directed towards the metering element (33).

4. The assembly according to any of the previous claims,
configured such that the sealing lip (81) is deflected when the metering element (33) is moved with respect to the storage chamber (15).

5. The assembly according to claim 4,
configured such that the sealing lip (81) is deflected in a distal direction (18) when the metering element (33) is moved in a distal direction (18), and wherein the sealing lip (81) is deflected in a proximal direction (19) when the metering element (33) is moved in a proximal direction (19).

6. The assembly according to any of the previous claims,
wherein the sealing member (31) comprises a main part (32), and wherein the at least one sealing lip (81) is connected to the main part.

7. The assembly according to any of the previous claims,
wherein the at least one sealing lip (81) is disposed circumferentially around the metering element (33).

8. The assembly according to any of the previous claims,
wherein the at least one sealing lip (81) is adapted and arranged for removing excess powdery substance (2) from the metering element (33) when the metering element (33) is moved along a dosing direction (18) with respect to the storage chamber (15).

9. The assembly according to any of the previous claims,
wherein the at least one sealing lip (81) is configured to seal the storage chamber (15).

10. The assembly according to any of the previous claims,
wherein the at least one sealing lip (81) comprises a flexible material.

11. The assembly according to any of the previous claims,
comprising a plurality of sealing lips (81a, 81b).

12. The assembly according to claim 11,
wherein the sealing member (31) comprises the plurality of sealing lips (81 a, 81 b).

13. The assembly according to any of claims 11 or 12,
wherein the sealing lips (81a, 81b) are arranged one above another in a direction along the longitudinal axis (mx) of the metering element (33).

14. The assembly according to any of claims 11 to 13,
wherein the sealing member (31) comprises a cavity (42) which is arranged between two adjacent sealing lips (81a, 81b).

15. Use of a sealing member (31) for mechanical cooperation with a metering element (33) of an inhalation device (1), wherein the sealing member (31) comprises an opening (20) which receives the metering element (33), and wherein the sealing member (31) comprises at least one sealing lip (81), **characterised in that** the sealing lip (81) is configured to be deflected in a direction corresponding to a moving direction of the metering element (33).

## Patentansprüche

1. Anordnung für eine Inhalationsvorrichtung (1), wobei die Anordnung Folgendes umfasst:
- eine Vorratskammer (15), die zum Halten einer Menge einer Substanz (2) konfiguriert ist,
- ein Dosierelement (33), das dazu konfiguriert ist, in einer Bewegungsrichtung bezüglich der Vorratskammer (15) bewegt zu werden,
und
- ein Dichtglied (31), das mechanisch mit dem Dosierelement (33) zusammenwirkt, wobei das Dichtglied (31) eine Öffnung (20) umfasst, die das Dosierelement (33) aufnimmt, und wobei das Dichtglied (31) eine Dichtlippe (81) umfasst, **dadurch gekennzeichnet, dass** die Dichtlippe in einer der Bewegungsrichtung des Dosiereiements (33) entsprechenden Richtung umlenkbar ist.

2. Anordnung nach Anspruch 1,
wobei ein Querschnitt der Dichtlippe (81) trapezförmig ist.

3. Anordnung nach Anspruch 2,
wobei das schmalere Ende des Trapezes zum Dosierelement (33) hin gerichtet ist.

4. Anordnung nach einem der vorhergehenden Ansprüche,
die so konfiguriert ist, dass die Dichtlippe (81) umgelenkt wird, wenn das Dosierelement (33) bezüglich der Vorratskammer (15) bewegt wird.

5. Anordnung nach Anspruch 4,
die so konfiguriert ist, dass die Dichtlippe (81) in eine distale Richtung (18) umgelenkt wird, wenn das Dosierelement (33) in eine distale Richtung (18) bewegt wird, und wobei die Dichtlippe (81) in eine proximale Richtung (19) umgelenkt wird, wenn das Dosierelement (33) in eine proximale Richtung (19) bewegt wird.

6. Anordnung nach einem der vorhergehenden Ansprüche,
wobei das Dichtglied (31) einen Hauptteil (32) umfasst und wobei die mindestens eine Dichtlippe (81) mit dem Hauptteil verbunden ist.

7. Anordnung nach einem der vorhergehenden Ansprüche,
wobei die mindestens eine Dichtlippe (81) umfangsmäßig um das Dosierelement (33) angeordnet ist.

8. Anordnung nach einem der vorhergehenden Ansprüche,
wobei die mindestens eine Dichtlippe (81) dazu geeignet und angeordnet ist, überschüssige pulverförmige Substanz (2) von dem Dosierelement (33) zu entfernen, wenn das Dosierelement (33) entlang einer Dosierrichtung (18) bezüglich der Vorratskammer (15) bewegt wird.

9. Anordnung nach einem der vorhergehenden Ansprüche,
wobei die mindestens eine Dichtlippe (81) dazu konfiguriert ist, die Vorratskammer (15) abzudichten.

10. Anordnung nach einem der vorhergehenden Ansprüche,
wobei die mindestens eine Dichtlippe (81) ein flexibles Material umfasst.

11. Anordnung nach einem der vorhergehenden Ansprüche, umfassend eine Vielzahl von Dichtlippen (81a, 81b).

12. Anordnung nach Anspruch 11,
wobei das Dichtglied (31) die Vielzahl von Dichtlippen (81a, 81b) umfasst.

13. Anordnung nach einem der Ansprüche 11 oder 12,
wobei die Dichtlippen (81a, 81b) in einer Richtung entlang der Längsachse (mx) des Dosierelements (33) übereinander angeordnet sind.

14. Anordnung nach einem der Ansprüche 11 bis 13,
wobei das Dichtglied (31) einen Hohlraum (42) umfasst, der zwischen zwei benachbarten Dichtlippen (81a, 81b) angeordnet ist.

15. Verwendung eines Dichtglieds (31) für das mechanische Zusammenwirken mit einem Dosierelement (33) einer Inhalationsvorrichtung (1), wobei das Dichtglied (31) eine Öffnung (20) umfasst, die das Dosierelement (33) aufnimmt, und wobei das Dichtglied (31) mindestens eine Dichtlippe (81) umfasst, **dadurch gekennzeichnet, dass** die Dichtlippe (81) dazu konfiguriert ist, in einer Richtung umgelenkt zu werden, die einer Bewegungsrichtung des Dosierelements (33) entspricht.

## Revendications

1. Ensemble pour un dispositif d'inhalation (1), l'ensemble comprenant :
- une chambre de stockage (15) configurée pour renfermer une quantité d'une substance (2),
- un élément de mesure (33) configuré pour être déplacé dans une direction de déplacement par rapport à la chambre de stockage (15), et
- un organe d'étanchéité (31) coopérant de manière mécanique avec l'élément de mesure (33), l'organe d'étanchéité (31) comprenant une ouverture (20) qui reçoit l'élément de mesure (33), et l'organe d'étanchéité (31) comprenant une lèvre d'étanchéité (81),
**caractérisé en ce que** la lèvre d'étanchéité peut être courbée dans une direction correspondant à la direction de déplacement de l'élément de mesure (33).

2. Ensemble selon la revendication 1,
dans lequel une section transversale de la lèvre d'étanchéité (81) présente la forme d'un trapèze.

3. Ensemble selon la revendication 2,
dans lequel l'extrémité étroite du trapèze est orientée en direction de l'élément de mesure (33).

4. Ensemble selon l'une quelconque des revendications précédentes, configuré de telle sorte que la lèvre d'étanchéité (81) soit courbée lorsque l'élément de mesure (33) est déplacé par rapport à la chambre de stockage (15).

5. Ensemble selon la revendication 4,
configuré de telle sorte que la lèvre d'étanchéité (81) soit courbée dans une direction distale (18) lorsque l'élément de mesure (33) est déplacé dans une direction distale (18), et dans lequel la lèvre d'étanchéité (81) est courbée dans une direction proximale (19) lorsque l'élément de mesure (33) est déplacé dans une direction proximale (19).

6. Ensemble selon l'une quelconque des revendications précédentes, dans lequel l'organe d'étanchéité (31) comprend une partie principale (32) et dans lequel la ou les lèvres d'étanchéité (81) sont raccordées à la partie principale.

7. Ensemble selon l'une quelconque des revendications précédentes, dans lequel la ou les lèvres d'étanchéité (81) sont disposées de manière circonférentielle autour de l'élément de mesure (33).

8. Ensemble selon l'une quelconque des revendications précédentes, dans lequel la ou les lèvres d'étanchéité (81) sont conçues et disposées pour éliminer un excès de substance pulvérulente (2) de l'élément de mesure (33) lorsque l'élément de mesure (33) est déplacé le long d'une direction de dosage (18) par rapport à la chambre de stockage (15).

9. Ensemble selon l'une quelconque des revendications précédentes, dans lequel la ou les lèvres d'étanchéité (81) sont configurées pour fermer hermétiquement la chambre de stockage (15).

10. Ensemble selon l'une quelconque des revendications précédentes, dans lequel la ou les lèvres d'étanchéité (81) comprennent un matériau souple.

11. Ensemble selon l'une quelconque des revendications précédentes, comprenant une pluralité de lèvres d'étanchéité (81 a, 81 b).

12. Ensemble selon la revendication 11,
dans lequel l'organe d'étanchéité (31) comprend la pluralité de lèvres d'étanchéité (81a, 81b).

13. Ensemble selon l'une quelconque des revendications 11 et 12, dans lequel les lèvres d'étanchéité (81a, 81 b) sont disposées les unes au-dessus des autres dans une direction le long de l'axe longitudinal (mx) de l'élément de mesure (33).

14. Ensemble selon l'une quelconque des revendications 11 à 13, dans lequel l'organe d'étanchéité (31) comprend une cavité (42) qui est disposée entre deux lèvres d'étanchéité (81 a, 81 b) adjacentes.

15. Utilisation d'un organe d'étanchéité (31) à des fins de coopération mécanique avec un élément de mesure (33) d'un dispositif d'inhalation (1), l'organe d'étanchéité (31) comprenant une ouverture (20) qui reçoit l'élément de mesure (33), et l'organe d'étanchéité (31) comprenant au moins une lèvre d'étanchéité (81),
**caractérisée en ce que** la lèvre d'étanchéité (81) est configurée pour être courbée dans une direction correspondant à une direction de déplacement de l'élément de mesure (33).
